# EUROPEAN PATENT APPLICATION

(11) **EP 1 361 283 A1**
(43) Date of publication of application: **12.11.2003**
(21) Application number: 02712305.8
(22) Date of filing: 13.02.2002
(51) Int. Cl.: C12Q 1/32, C12Q 1/40, C12Q 1/50, C12Q 1/54, C12Q 1/61

(54) **NOVEL ASSAY METHOD**

(30) Priority: 14.02.2001 JP 2001036863
(71) Applicant: International Reagents Corporation, Kobe-shi Hyogo 651-2271 (JP)
(72) Inventor: KISHI, Koji c/o Research & Development Center, Nishi-ku Kobe-shi Hyogo 651-2241 (JP); YAMASHITA, Kazuaki c/o Res. & Development Center, Nishi-ku Kobe-shi Hyogo 651-2241 (JP)
(74) Representative: HOFFMANN - EITLE
(86) International application number: JP0201173
(87) International publication number: WO02064819

(57) **Abstract**

It is intended to provide novel assay method whereby accurate data can be obtained in assaying a test substance in a biological sample with the use of a reaction system enabling the formation of 6-phosphogluconic acid. It is noted that 6-phosphogluconic acid is the final product in a reaction system of assaying glucose by using a coenzyme and that 6PGDH(6-phosphogluconate dehydrogenase) in blood cells reacts using NADP as a coenzyme to thereby results in errors in the assay data. In assaying a test substance in a biological sample with the use of a reaction system enabling the formation of 6-phosphogluconic acid, the above object can be achieved by excluding 6-phosphogluconate from the reaction system.

## Description

### TECHNICAL FIELD

The present invention relates to a measuring method avoiding measurement errors caused by a hemolytic constituent contained in the specimen when a subject matter is measured using a measurement reaction system which utilizes an enzyme reaction for a biological sample.

### BACKGROUND ART

In the field of clinical examination, a method in which a subject constituent is quantitatively measured by receiving variation of amount of coenzymes (NAD, NADP, NADH, NADPH, Thio-NAD, Thio-NAD, Thio-HADH, Thio-NADP, Thio-NADPH) as variation of absorbance is often used. Serum is generally used as a specimen but, since there are cases where physical impacts are given to blood cells when blood is taken to obtain serum or blood cells burst because of a human mis-operation, hemolysis may be induced. When blood is taken from a patient having a hemolytic disease, hemolysis may be recognized because of the disease. Furthermore, recently, measurements in which the entire blood is used to reduce the time necessary for an examination are attempted and hemolysis may also be recognized for such specimens.

When a blood cell has burst and results in the state of hemolysis, such enzymes as myokinase (adenylate kinase (hereinafter referred to as "MK")), glucose-6-phosphate dehydrogenase (hereinafter referred to as "G6PDH") etc. being the constituents of a red blood cell get mixed with serum as a result. Since measured values are varied by the influence of these enzymes depending on the item of clinical examination, countermeasures against each enzyme have been taken. For example, it is reported to use a a glycolysis preventive comprising a mixture of NaF, MgCl₂, NaH₂PO₄ and an anticoaglant in order to avoid measurement errors caused by hemolysis when a glucose constituent is measured (JP Publication of Unexamined Patent No. H05-126834) and AP₅A is reported to be effective for MK activity.

However, the measurement errors caused by the constituents in red blood cells contained in a specimen, i.e., a problem of hemolysis interference in the measurement in clinical examinations utilizing enzyme reactions can not be said yet to be solved.

### DISCLOSURE OF THE INVENTION

The object of the invention is to provide a measuring method to obtain correct measured values when the subject matter is measured using a reaction system capable of producing 6-phosphogluconate for a biological sample.

As a result of studying diligently to solve the above consideration, the inventor noted that the end product of a reaction system for measuring glucose using a coenzyme is 6-phosphogluconate (hereinafter abbreviated to "6PG") and found that 6-PG-dehydrogenase (hereinafter abbreviated to "6PGDH" ) reacts utilizing NADP as a coenzyme and a difference is produced between correct and erred values for values of 6PG as the result of a glucose measurement system.

Then, in a reaction system for measuring glucose using a coenzyme, the inventor found a method for obtaining correct measured values of glucose by placing 6PGDH outside of the reaction system. Conventionally, there has been no report of the influence of 6PGDH activity when hemolysis occurs and the inventor has found it first. It is considered that the influence of hemolysis has been considered to be caused by MK activity, glucose in blood cells and phosphorus acid, and 6PGDH has not been noted.

That is, the present invention provides:
1. A subject matter measuring method wherein when a subject matter is measured using a reaction system capable of producing 6-phosphogluconate for a biological sample, 6-phosphogluconate dehydrogenase is placed outside of the reaction system;
2. The subject matter measuring method according to item 1, wherein means for placing 6-phosphogluconate dehydrogenase outside of the reaction system is to use a coenzyme which does not react with 6-phosphogluconate dehydrogenase in the reaction system;
3. The subject matter measuring method according to item 2, wherein NAD or Thio-NAD is used as the coenzyme which does not react with 6-phosphogluconate dehydrogenase;
4. The subject matter measuring method according to item 1, wherein a 6-phosphogluconate dehydrogenase activation inhibitor is used as the means for placing 6-phosphogluconate dehydrogenase outside of the reaction system;
5. The subject matter measuring method according to item 1, wherein an anti-6-phosphogluconate dehydrogenase antibody is used as the means for placing 6-phosphogluconate dehydrogenase outside of the reaction system;
6. The subject matter measuring method according to any one of items 1 to 5, wherein further lactate dehydrogenase is placed outside of the reaction system;
7. The subject matter measuring method according to item 6, wherein the means for placing lactate dehydrogenase outside of the reaction system is to contain oxalic acid, oxamic acid or their salts therein;
8. The subject matter measuring method according to any one of items 1 to 7, wherein the subject matter is a substance which is measurable by using a reaction system capable of producing glucose;
9. The subject matter measuring method according to any one of items 1 to 8, wherein the subject matter is one or more substances selected from hexose, neutral fat, inorganic phosphorus, creatine kinase or its isozyme, or amylase or its isozyme;
10. A method for avoiding hemolysis interference wherein 6-phosphogluconate dehydrogenase generated by hemolysis is placed outside of the reaction system; and
11. A reagent composition comprising reagents necessary for the method according to any one of claims 1 to 9 in the form of a kit or comprising one of the reagents.

### BRIEF DESCRIPTION OF DRAWINGS

Fig. 1 illustrates a reaction system capable of producing 6PG for measurement of glucose;
Fig. 2 illustrates a reaction system capable of producing 6PG for measurement of neutral fat;
Fig. 3 shows variation of absorbance of hemolysate in an NAD reaction system (Example 1);
Fig. 4 shows variation of absorbance of hemolysate in an NADP reaction system (Example 1);
Fig. 5 shows variation of measured values of glucose (Example 2); and
Fig. 6 shows variation of measured values of neutral fat (Example 3).

### BEST MODE FOR CARRYING OUT THE INVENTION

In the invention, as the case where a subject matter is measured for a biological specimen using a reaction system capable of producing 6PG, a reaction system for measuring hexose or phosphoric-acidificated hexose using coenzymes is exemplified. Hexose refers to 6-carbon sugars and its representative one is glucose. As the items of the clinical examination for measurement using such a measurement system, glucose (GLU), neutral fat (TG), inorganicphosphorus (IP), creatine kinase (CPK), sucrose, inulin, ureanitrogen (BUN) and creatinine are exemplified.

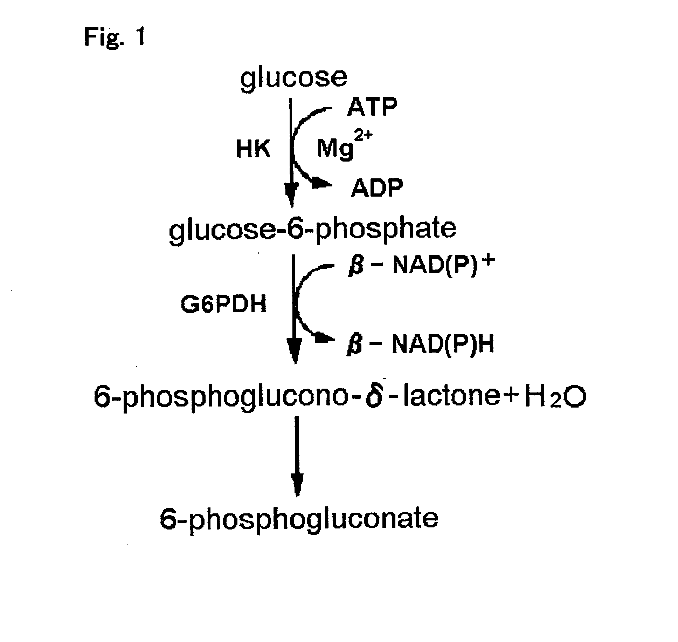

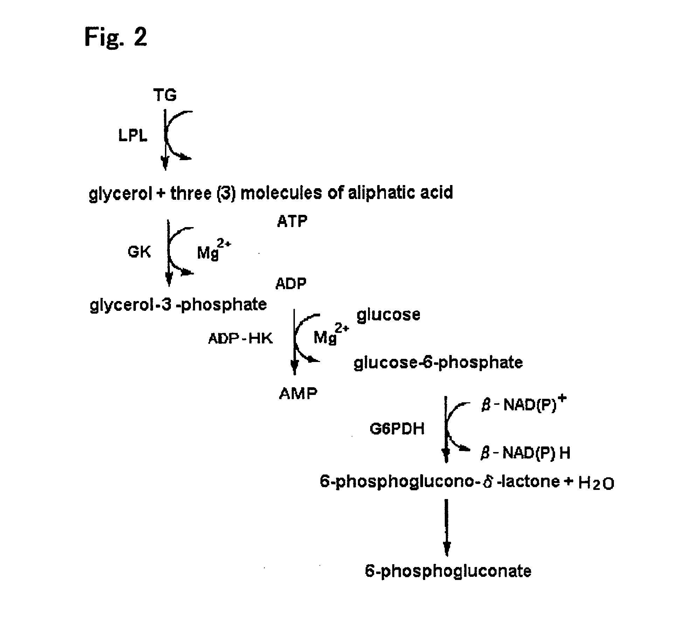

For example, as shown in Fig. 1 and Fig. 2, the end product in the case where a reaction system for producing glucose is used is 6PG when glucose or neutral fat is measured.

For a reaction system for measuring hexose or phosphoric-acidificated hexose using coenzymes, there is a phenomenon that difference is produced between the correct and erred values for measured values when hemolysis occurs and this phenomenon is called hemolysis interference.

This phenomenon was considered that, because of hemolysis, 6PGDH in blood cells reacts with 6PG using NADP as a coenzyme in the measurement system and, consequently, hemolysis interference occurs since the correct measured value of 6PG can not be obtained. The invention is to have found that 6PGDH produced when hemolysis occurs causes the interference action and, furthermore, that, in the above measurement system, the interference action caused by hemolysis can be avoided by placing 6PGDH outside of the reaction system.

As means for placing 6PGDH outside of the reaction system, to use a coenzyme which does not react with 6PGDH in the reaction system, to use an activity preventive against 6PGDH, to add an antibody of 6PGDH or to remove the immunity of 6PGDH etc. are exemplified. Furthermore, the inventor has found out that 6PGDH reacts using NADP as a coenzyme but it does not recognize NAD as a coenzyme, and found that 6PGDH can consequently be placed outside of the reaction system for measurement by constructing the reaction system using NAD. Then, the inventor has invented a novel measuring method that avoids the influence of hemolysis.

Yet furthermore, NAD and NADH are influenced by a dehydrogenase, especially lactate dehydrogenase (hereinafter referred to as "LDH"; NAD-depedent) in the specimen but the influence of LDH can be avoided completely by presence of oxalic acid, oxamic acid or their salts in the reaction system. Therefore, this phenomenon can be combined with the measuring method.

Yet furthermore, in the reaction system for measuring hexose or phosphoric-acidificated hexose using a coenzyme of the invention, one or more enzyme ( s ) or sugar(s) selected from HK, G6PDH, hexose, G6P can be used being combined with the coenzyme. Methods for measuring glucose with these enzyme approaches are widely known.

### [Examples]

The invention will be described in detail as follows referring to examples but the invention is not limited to these examples.

### [Example 1]

After taking the blood with a blood-collecting tube, human red blood cells were obtained by centrifuging the blood at 3,000rpm. The blood cells were frozen at -20°C and melted at a room temperature. These operations caused the blood cells to burst and hemolysis constituents were obtained. The constituents were diluted with physiological salt solution to a hemoglobin concentration of 500mg/dL. Then, the reagents described as follows were prepared.

A reagent solution in which 2.0mM of NADP and 1.0mM of 6PG were solved into 0.1M of triethanolamine buffer solution (pH 7.5) (Reagent A) and a reagent solution in which 2.0mM of NAD and 1.0mM of 6PG were solved into 0.1M of triethanolamine buffer solution (pH 7.5) (Reagent B) were prepared.

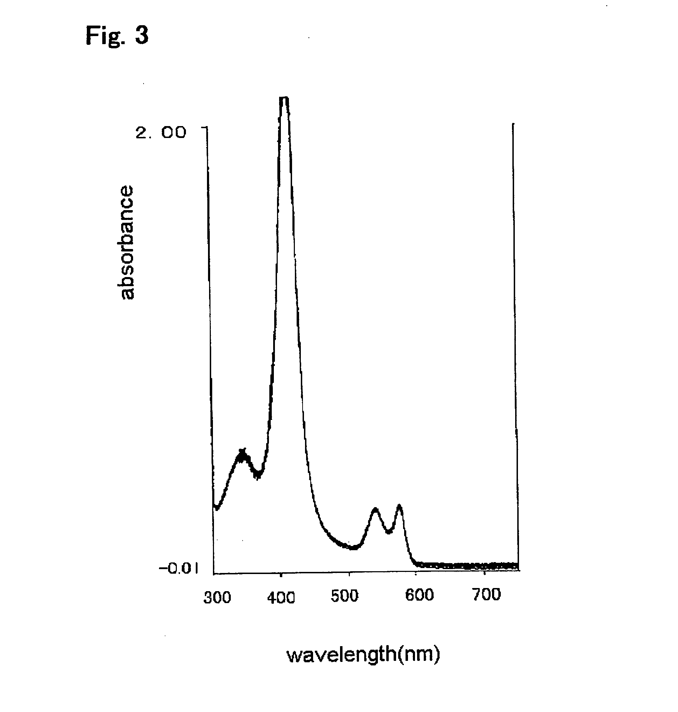

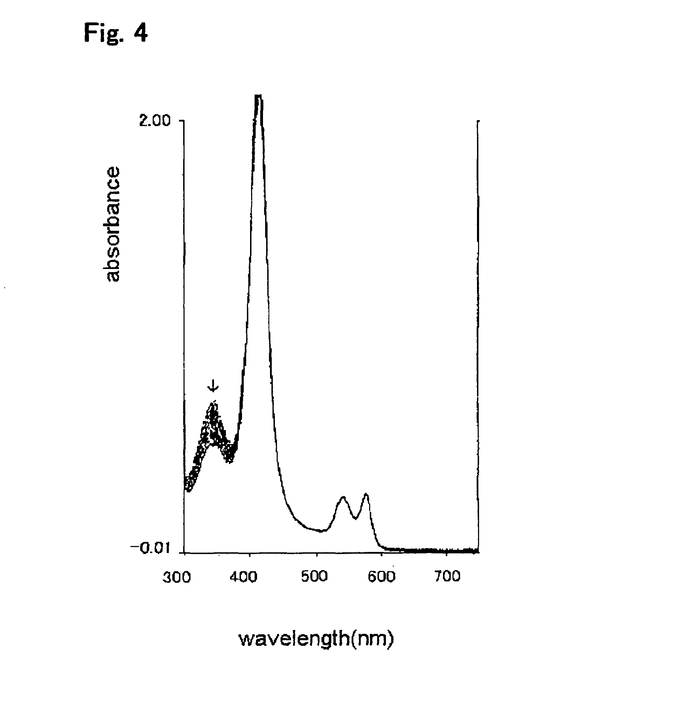

After heating respectively Reagent A and Reagent B at 37°C for five (5) minutes, 15 µL of hemolyzed specimen was added to each of them and their absorbance at 340-750 nm was measured every one (1) minute. As a result of measuring this hemolyzed solution, as shown in Fig. 3, no increase of absorbance was recognized for the reagent in which NAD was used. As shown in Fig. 4, absorbance at 340nm for NADP was increased for the case where reaction was caused by using NADP as a coenzyme. As a result, it was suggested that hemolysis interference could be avoided by using NAD.

### [Example 2]

The following reagents were prepared and used for measurement of glucose (GLU).

### [First Reagent]

Tris: 100mM
HK: 3.5U/mL
G6PDH: 5.0U/mL
β-NAD: 3mM
magnesium acetate: 10mM
pH 6.0

### [Second Reagent]

Tris: 200mM
ATP: 6mM
pH 9.0

Reagents using β-NADP instead of β-NAD in the above compositions were also prepared.

### [Operation]

210µL of first reagent and 70µL of Second Reagent were added to 5µL of specimen. End point was assayed for the specimen mixture in a Hitachi model-7170 automatic analyzer under the condition of main wavelength to be at 340nm and glucose concentration was calculated from a working curve produced in advance.

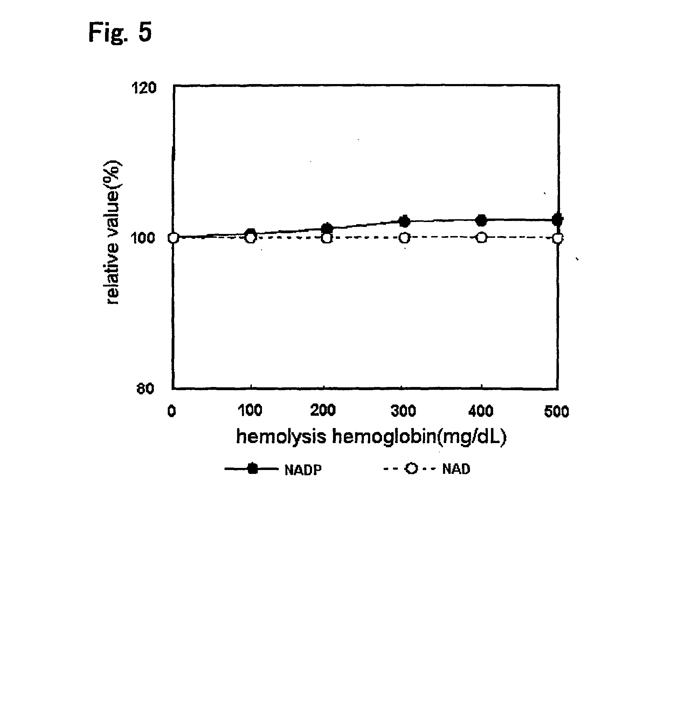

Some specimen mixtures were prepared such that the hemoglobin concentration in each specimen mixture was 0, 100, 200, 300, 400 and 500mg/dL respectively and the concentration of human serum constituents was same in all specimen mixtures. As shown in Fig. 5, for Reagent A using NADP as a coenzyme, glucose measurements increased almost depending on the hemoglobin concentration but, for Reagent using NAD, no increase of glucose measurements was observed and no difference between correct values and erred values was produced. Glucose concentration was 80mg/dL when hemoglobin concentration was 0mg/dL.

### [Example 3]

The following reagents were prepared and used for measurement of neutral fat (TG).

### [First Reagent]

Bicine: 50mM
Potassium-chloride: 100mM
Magnesium chloride: 10mM
Nonion A-10R: 0.5%
Triton X-100: 0.2%
Sodium azide: 0.1%
PEP: 4.0mM
ATP: 3.0mM
G6PDH: 4.5U/mL
PK: 3.0U/mL
GK: 3.0U/mL
pH: 8.5

### [Second Reagent]

MES: 50mM
Oxalic acid: 100mM
Glucose: 80mM
Triton X-100: 0.25%
β-NAD: 7.0mM
Sodium azide: 0.1%
ADP-HK: 10.0U/mL
Lipase: 1500U/mL
pH: 6.5

Reagents using β-NADP instead of β -NAD in the above compositions were also prepared.

The operation for measurement was carried out in the same way as the measurement of glucose and neutral fat was measured.

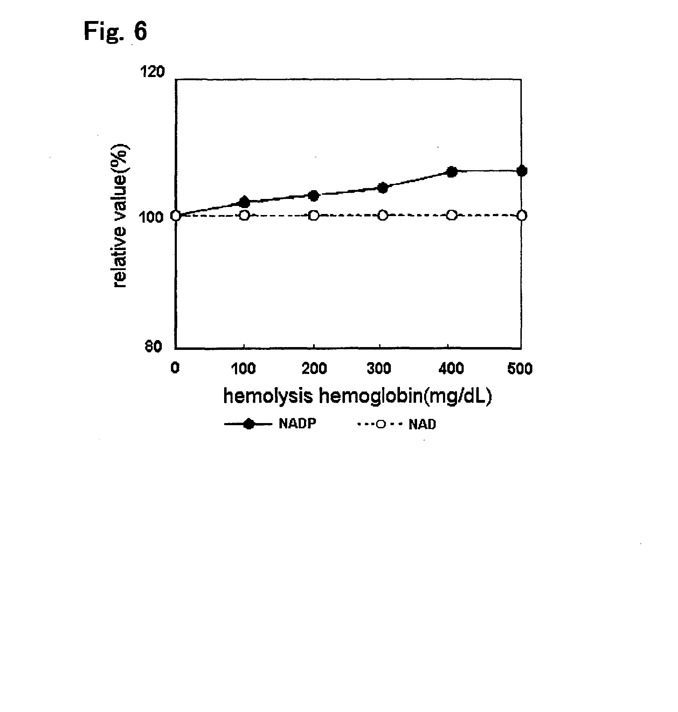

Some specimen mixtures were prepared such that the hemoglobin concentration in each specimen mixture was 0, 100, 200, 300, 400 and 500mg/dL respectively and the concentration of human serum constituents was same in all specimen mixtures. As shown in Fig. 6, for Reagent B using NADP as a coenzyme, neutral fat measurements increased almost depending on the hemoglobin concentration but, for Reagent using NAD, no increase of neutral fat measurements was observed and no difference between correct values and erred values was produced. Neutral fat concentration was 95mg/dL when hemoglobin concentration was 0mg/dL.

### INDUSTRIAL APPLICABILITY

As set forth hereinabove, when a subject matter is measured using a reaction system capable of producing 6-phosphogluconate for a biological sample, it has become possible by placing 6-phosphate dehydrogenase outside of the reaction system to avoid hemolysis interference and obtain correct measurements for the subject matter.

## Claims

1. A subject matter measuring method wherein when a subject matter is measured using a reaction system capable of producing 6-phosphogluconate for a biological sample, 6-phosphogluconate dehydrogenase is placed outside of the reaction system.

2. The subject matter measuring method according to claim 1, wherein means for placing 6-phosphogluconate dehydrogenase outside of the reaction system is to use a coenzyme which does not react with 6-phosphogluconate dehydrogenase in the reaction system.

3. The subject matter measuring method according to claim 2, wherein NAD or Thio-NAD is used as the coenzyme which does not react with 6-phosphogluconate dehydrogenase.

4. The subject matter measuring method according to claim 1, wherein a 6-phosphogluconate dehydrogenase activation inhibitor is used as the means for placing 6-phosphogluconate dehydrogenase outside of the reaction system.

5. The subject matter measuring method according to claim 1, wherein an anti-6-phosphogluconate dehydrogenase antibody is used as the means for placing 6-phosphogluconate dehydrogenase outside of the reaction system.

6. The subject matter measuring method according to any one of claims 1 to 5, wherein further lactate dehydrogenase is placed outside of the reaction system.

7. The subject matter measuring method according to claim 6, wherein the means for placing lactate dehydrogenase outside of the reaction system is to contain oxalic acid, oxamic acid or their salts therein.

8. The subject matter measuring method according to any one of claims 1 to 7, wherein the subject matter is a substance which is measurable by using a reaction system capable of producing glucose.

9. The subject matter measuring method according to any one of claims 1 to 8, wherein the subject matter is one or more substances selected from hexose, neutral fat, inorganic phosphorus, creatine kinase or its isozyme, or amylase or its isozyme.

10. A method for avoiding hemolysis interference wherein 6-phosphogluconate dehydrogenase generated by hemolysis is placed outside of the reaction system.

11. A reagent composition comprising reagents necessary for the method according to any one of claims 1 to 9 in the form of a kit or comprising one of the reagents.
